# EUROPEAN PATENT SPECIFICATION

(11) **EP 4 482 535 B1**
(45) Date of publication and mention of the grant of the patent: **08.04.2026**
(21) Application number: 23709918.9
(22) Date of filing: 16.02.2023
(51) Int. Cl.: A61L 2/00, A61L 2/10, A61L 9/20, F24F 8/22, A61L 2/24

(54) **UV EMITTING UPPER AIR DISINFECTION DEVICE**
UV-EMITTIERENDE VORRICHTUNG ZUR DESINFEKTION DER OBEREN LUFT
DISPOSITIF DE DÉSINFECTION DE L'AIR SUPÉRIEUR ÉMETTANT DES UV

(30) Priority: 24.02.2022 EP 22158542
(43) Date of publication of application: 01.01.2025
(73) Proprietor: Signify Holding B.V., 5656 AE Eindhoven (NL)
(72) Inventor: VAN BOMMEL, Ties, 5656 AE Eindhoven (NL); HIKMET, Rifat, Ata, Mustafa, 5656 AE Eindhoven (NL)
(74) Representative: Verweij, Petronella Daniëlle
(86) International application number: PCT/EP2023/053850
(87) International publication number: WO 2023/161110

(56) References cited:
- WO-A1-03/027569
- KR-B1- 100 998 473
- US-A1- 2014 105 784

## Description

### FIELD OF THE INVENTION

The invention relates to a light generating system and to a method for treating a gas, such as air, or a surface.

### BACKGROUND OF THE INVENTION

Ultraviolet treatment devices are known in the art. US2014/0105784, for instance, describes a light source for use in a treatment device for treating a fluid, solid or surface. The light source includes a light emitting diode that emits a first component of ultraviolet (UV) light, and a UV laser light source that emits a second component of UV light having a peak wavelength different from a peak wavelength of the first component of UV light. The first component of UV light and the second component of UV light are applied to treat the fluid or surface. The UV laser light source may include a laser light source and a frequency doubling component that receives light from the laser light source and converts the light to the second component of UV light. A treatment device includes the described light source and a container containing the fluid, or a solid surface, to be treated.

WO03/027569A discloses an edge-lit illumination device with a polarized light-emitting waveguide that has polarization-selective outcoupling means including at least a volume hologram for selectively diffracting waveguided light towards the exit surface of the waveguide with high contrast and efficiency. The light emitted by the waveguide is selectively emitted to one side thereof, is highly polarized and highly collimated, and homogeneously distributed across the exit surface.

### SUMMARY OF THE INVENTION

UV light has been used for disinfection for over 100 years. Wavelengths between about 190 nm and 300 nm may be strongly absorbed by nucleic acids, which may result in defects in an organism's genome. This may be desired for inactivating (killing), bacteria and viruses, but may also have undesired side effects for humans. Therefore, the selection of wavelength of radiation, intensity of radiation and duration of irradiation may be limited in environments where people may reside such as offices, public transport, cinema's, restaurants, shops, etc., thus limiting the disinfection capacity. Especially in such environments, additional measures of disinfection may be advantageous to prevent the spread of bacteria and viruses such as influenza or novel (corona) viruses like COVID-19, SARS, and MERS.

It appears desirable to produce systems, that provide alternative ways for air treatment, such as disinfection. Further, existing systems for disinfection may not easily be implemented in existing infrastructure, such as in existing buildings like offices, hospitality areas, etc. and/or may not easily be able to serve larger spaces. This may again increase the risk of contamination. Further, incorporation in HVAC systems may not lead to desirable effects and appears to be relatively complex. Further, existing systems may not be efficient, or may be relatively bulky, and may also not easily be incorporated in functional devices, such as e.g. luminaires.

Other disinfection systems may use one or more anti-microbial and/or antiviral means to disinfect a space or an object. Examples of such means may be chemical agents which may raise concerns. For instance, the chemical agents may also be harmful for people and pets.

In embodiments, the disinfecting light, may especially comprise ultraviolet (UV) radiation (and/or optionally violet radiation), i.e., the light may comprise a wavelength selected from the ultraviolet wavelength range (and/or optionally the violet wavelength range). However, other wavelengths are herein not excluded. The ultraviolet wavelength range is defined as light in a wavelength range from 100 to 380 nm and can be divided into different types of UV light / UV wavelength ranges (Table 1). Different UV wavelengths of radiation may have different properties and thus may have different compatibility with human presence and may have different effects when used for disinfection (Table 1).

**Table 1: Properties of different types of UV, violet, and NIR wavelength light**

| Name | Short name | Wavelength (nm) | (Relative) sterilization effectiveness | | Safe Radiation | Vitamin D generation | Ozone generation |
|---|---|---|---|---|---|---|---|
| | | | Bacteria | Viruses | | | |
| Near Infrared (deep red) | NIR | 780-950 | + | +/- | +++ | | |
| Violet | V | 380-420 | +/- | - | + | | |
| Ultra violet A | UV-A | 315-380 | + | - | + | | |
| Ultraviolet B | UV-B | 280-315 | + | +/- | +/- | + | |
| Near ultraviolet C | Near UV-C | 230-280 | ++ | ++ | - | | |
| Far ultraviolet C | Far UV-C | 190-230 | +++ | +++ | + | | +/- |
| Extreme ultraviolet C | Extreme UV-C | 100-190 | +++ | +++ | - | | + |

Each UV type / wavelength range may have different benefits and/or drawbacks. Relevant aspects may be (relative) sterilization effectiveness, safety (regarding radiation), and ozone production (as result of its radiation). Depending on an application a specific type of UV light or a specific combination of UV light types may be selected and provides superior performance over other types of UV light. UV-A may be (relatively) safe and may inactivate (kill) bacteria, but may be less effective in inactivating (killing) viruses. UV-B may be (relatively) safe when a low dose (i.e. low exposure time and/or low intensity) is used, may inactivate (kill) bacteria, and may be moderately effective in inactivating (killing) viruses. UV-B may also have the additional benefit that it can be used effectively in the production of vitamin D in a skin of a person or animal. Near UV-C may be relatively unsafe, but may effectively inactivating, especially kill bacteria and viruses. Far UV-C may also be effective in inactivating (killing) bacteria and viruses, but may be (relatively to other UV-C wavelength ranges) (rather) safe. Far-UV light may generate some ozone which may be harmful for human beings and animals. Extreme UV-C may also be effective in inactivating (killing) bacteria and viruses, but may be relatively unsafe. Extreme UV-C may generate ozone which may be undesired when exposed to human beings or animals. **In** some application ozone may be desired and may contribute to disinfection, but then its shielding from humans and animals may be desired. Hence, in the table "+" for ozone production especially implies that ozone is produced which may be useful for disinfection applications, but may be harmful for humans / animals when they are exposed to it. Hence, in many applications this "+" may actually be undesired while in others, it may be desired. The types of light indicated in above table may in embodiments be used to sanitize air and/or surfaces.

The terms "inactivating" and "killing" with respect to a virus may herein especially refer to damaging the virus in such a way that the virus can no longer infect and/or reproduce in a host cell, i.e., the virus may be (essentially) harmless after inactivation or killing.

Hence, in embodiments, the light may comprise a wavelength in the UV-A range. In further embodiments, the light may comprise a wavelength in the UV-B range. In further embodiments, the light may comprise a wavelength in the Near UV-C range. In further embodiments, the light may comprise a wavelength in the Far UV-C range. In further embodiments, the light may comprise a wavelength in the extreme UV-C range. The Near UV-C, the Far UV-C and the extreme UV-C ranges may herein also collectively be referred to as the UV-C range. Hence, in embodiments, the light may comprise a wavelength in the UV-C range. In other embodiments, the light may comprise violet radiation.

Hence, light or radiation described herein may also be indicated as disinfection light.

Lighting system for providing disinfection light may suffer from the fact that radiation may also be provided to less desired parts of spaces. For instance, it may be desirable to prevent situations that radiation is potentially harmful to humans, e.g. after long exposure.

Hence, it is an aspect of the invention to provide an alternative light generating system (and method for treating gas or a surface in a space), which preferably further at least partly obviates one or more of above-described drawbacks. The present invention may have as object to overcome or ameliorate at least one of the disadvantages of the prior art, or to provide a useful alternative.

According to a first aspect, the invention provides a light generating system ("system") comprising (i) n1 light generating devices and (ii) optics. Especially, the n1 light generating devices may be configured to generate device light having a wavelength selected from the wavelength range of 100 to 380 nm. Especially, n1 is at least 1. In specific embodiments, n1≥2. In further specific embodiments, the n1 light generating devices may comprise one or more laser devices. Especially, in embodiments the n1 light generating devices and optics may be configured to generate k1 beams of (beam-shaped) device light. In specific embodiments, each beam may have a first beam angle (θ1) and perpendicular thereto a second beam angle (θ2). Especially, the first beam angle (θ1) and the second beam angle (θ2) may be defined by a full width half maximum of the beam. In specific embodiments, the first beam angle (θ1) and the second beam angle (θ2) differ, especially in that one is larger than a predetermined value and the other is smaller than the predetermined value. Especially, in specific embodiments the predetermined value may be selected from the range of 1.5-5°, such as 2-5°. In embodiments, one of the first beam angle (θ1) and the second beam angle (θ2) may be selected from the range of at least 5° and the other one of the first beam angle (θ1) and the second beam angle (θ2) may be selected from the range of at maximum 2°. Further, k1 may be at least 1; more especially in embodiments k1≥2. In embodiments, each k1 beam has an optical axis (Ok). Further, in embodiments the light generating system may especially be configured to generate system light comprising at least one beam of device light. Hence, the invention provides in embodiments a light generating system comprising n1 light generating devices and optics, wherein: (A) the n1 light generating devices are configured to generate device light having a wavelength selected from the wavelength range of 100 to 380 nm; wherein n1≥2; wherein the n1 light generating devices comprise one or more laser devices; (B) the n1 light generating devices and optics are configured to generate k1 beams of (beam-shaped) device light wherein each beam has a first beam angle (θ1) and perpendicular thereto a second beam angle (θ2), wherein the first beam angle (θ1) and the second beam angle (θ2) are defined by a full width half maximum of the beam, wherein one of the first beam angle (θ1) and the second beam angle (θ2) is selected from the range of at least 5° and the other one of the first beam angle (θ1) and the second beam angle (θ2) is selected from the range of at maximum 2°; wherein k1≥2; and (C) the light generating system is configured to generate system light comprising at least one beam of device light.

With such system beams may be provided which may (essentially horizontally) skim just below a ceiling and may thus in general not be intercepted by people. Hence, with the present invention disinfection may be executed in a relatively safe way. Further, with the invention lamella, which may absorb light, are not necessarily used. Further, it may be possible to steer beams in specific embodiments. Further, with the present invention a superior collimation which may be obtained, which may not be obtainable with LEDs. Further, a superior spatial light distribution i.e. aspect ratio of vertical vs horizontal beam may be obtained, which may (also) not be obtainable with LEDs. Further, using frequency doubling, or other upconversion schemes, efficiency may be relatively high compared to short wavelength UV light sources, such as UV-C LEDs or lasers especially to low UVC wavelength light is much more efficient than LEDs, and relatively short wavelength UVC may even be difficult to achieve at all with solid state lasers or LEDs. Further, with the present invention an UV emitting upper air disinfection device may be provided.

As indicated above, the light generating system may comprise one or more light generating devices, especially at least two light generating devices. Hence, light generating system comprising n1 light generating devices, wherein in specific embodiments n1≥2.

A light generating device may especially be configured to generate device light. Especially, the light generating device may comprise a light source. The light source may especially configured to generate light source light. In embodiments, the device light may essentially consist of the device light. In other embodiments, the device light may essentially consist of converted light source light. In yet other embodiments, the device light may comprise (unconverted) light source light and converted light source light. Light source light may be converted with a luminescent material into luminescent material light and/or with an upconverter into upconverted light (see also below). The term "light generating device" may also refer to a plurality of light generating devices which may provide device light having essentially the same spectral power distributions. In specific embodiments, the term "light generating device" may also refer to a plurality of light generating devices which may provide device light having different spectral power distributions.

The term "light source" may in principle relate to any light source known in the art. It may be a conventional (tungsten) light bulb, a low pressure mercury lamp, a high pressure mercury lamp, a fluorescent lamp, a LED (light emissive diode). In a specific embodiment, the light source comprises a solid state LED light source (such as a LED or laser diode (or "diode laser")). The term "light source" may also relate to a plurality of light sources, such as 2-200 (solid state) LED light sources. Hence, the term LED may also refer to a plurality of LEDs. Further, the term "light source" may in embodiments also refer to a so-called chips-on-board (COB) light source. The term "COB" especially refers to LED chips in the form of a semiconductor chip that is neither encased nor connected but directly mounted onto a substrate, such as a PCB. Hence, a plurality of light emitting semiconductor light source may be configured on the same substrate. In embodiments, a COB is a multi LED chip configured together as a single lighting module.

The light source may have a light escape surface. Referring to conventional light sources such as light bulbs or fluorescent lamps, it may be outer surface of the glass or quartz envelope. For LED's it may for instance be the LED die, or when a resin is applied to the LED die, the outer surface of the resin. In principle, it may also be the terminal end of a fiber. The term escape surface especially relates to that part of the light source, where the light actually leaves or escapes from the light source. The light source is configured to provide a beam of light. This beam of light (thus) escapes from the light exit surface of the light source.

Likewise, a light generating device may comprise a light escape surface, such as an end window. Further, likewise a light generating system may comprise a light escape surface, such as an end window.

The term "light source" may refer to a semiconductor light-emitting device, such as a light emitting diode (LEDs), a resonant cavity light emitting diode (RCLED), a vertical cavity laser diode (VCSELs), an edge emitting laser, etc... The term "light source" may also refer to an organic light-emitting diode (OLED), such as a passive-matrix (PMOLED) or an active-matrix (AMOLED). In a specific embodiment, the light source comprises a solid-state light source (such as a LED or laser diode). In an embodiment, the light source comprises a LED (light emitting diode). The terms "light source" or "solid state light source" may also refer to a superluminescent diode (SLED).

The term LED may also refer to a plurality of LEDs.

The term "light source" may also relate to a plurality of (essentially identical (or different)) light sources, such as 2-2000 solid state light sources. In embodiments, the light source may comprise one or more micro-optical elements (array of micro lenses) downstream of a single solid-state light source, such as a LED, or downstream of a plurality of solid-state light sources (i.e. e.g. shared by multiple LEDs). In embodiments, the light source may comprise a LED with on-chip optics. In embodiments, the light source comprises a pixelated single LEDs (with or without optics) (offering in embodiments on-chip beam steering).

In embodiments, the light source may be configured to provide primary radiation, which is used as such, such as e.g. a blue light source, like a blue LED, or a green light source, such as a green LED, and a red light source, such as a red LED. Such LEDs, which may not comprise a luminescent material ("phosphor") may be indicated as direct color LEDs.

In other embodiments, however, the light source may be configured to provide primary radiation and part of the primary radiation is converted into secondary radiation. Secondary radiation may be based on conversion by a luminescent material. The secondary radiation may therefore also be indicated as luminescent material radiation. The luminescent material may in embodiments be comprised by the light source, such as a LED with a luminescent material layer or dome comprising luminescent material. Such LEDs may be indicated as phosphor converted LEDs or PC LEDs (phosphor converted LEDs). In other embodiments, the luminescent material may be configured at some distance ("remote") from the light source, such as a LED with a luminescent material layer not in physical contact with a die of the LED. Hence, in specific embodiments the light source may be a light source that during operation emits at least light at wavelength selected from the range of 380-470 nm. However, other wavelengths may also be possible. This light may partially be used by the luminescent material.

In embodiments, the light generating device may comprise a luminescent material. In embodiments, the light generating device may comprise a PC LED. In other embodiments, the light generating device may comprise a direct LED (i.e. no phosphor). In embodiments, the light generating device may comprise a laser device, like a laser diode. In embodiments, the light generating device may comprise a superluminescent diode. Hence, in specific embodiments, the light source may be selected from the group of laser diodes and superluminescent diodes. In other embodiments, the light source may comprise an LED.

The light source may especially be configured to generate light source light having an optical axis (O), (a beam shape,) and a spectral power distribution. The light source light may in embodiments comprise one or more bands, having band widths as known for lasers.

The term "light source" may (thus) refer to a light generating element as such, like e.g. a solid state light source, or e.g. to a package of the light generating element, such as a solid state light source, and one or more of a luminescent material comprising element and (other) optics, like a lens, a collimator. A light converter element ("converter element" or "converter") may comprise a luminescent material comprising element. For instance, a solid state light source as such, like a blue LED, is a light source. A combination of a solid state light source (as light generating element) and a light converter element, such as a blue LED and a light converter element, optically coupled to the solid state light source, may also be a light source (but may also be indicated as light generating device). Hence, a white LED is a light source (but may e.g. also be indicated as (white) light generating device).

The term "light source" herein may also refer to a light source comprising a solid state light source, such as an LED or a laser diode or a superluminescent diode.

The "term light source" may (thus) in embodiments also refer to a light source that is (also) based on conversion of light, such as a light source in combination with a luminescent converter material. Hence, the term "light source" may also refer to a combination of a LED with a luminescent material configured to convert at least part of the LED radiation, or to a combination of a (diode) laser with a luminescent material configured to convert at least part of the (diode) laser radiation.

In embodiments, the term "light source" may also refer to a combination of a light source, like a LED, and an optical filter, which may change the spectral power distribution of the light generated by the light source. Especially, the "term light generating device" may be used to address a light source and further (optical components), like an optical filter and/or a beam shaping element, etc.

The phrases "different light sources" or "a plurality of different light sources", and similar phrases, may in embodiments refer to a plurality of solid-state light sources selected from at least two different bins. Likewise, the phrases "identical light sources" or "a plurality of same light sources", and similar phrases, may in embodiments refer to a plurality of solid-state light sources selected from the same bin.

The term "solid state light source", or "solid state material light source", and similar terms, may especially refer to semiconductor light sources, such as a light emitting diode (LED), a diode laser, or a superluminescent diode.

Especially, the n1 light generating devices comprise laser light sources.

The term "laser light source" especially refers to a laser. Such laser may especially be configured to generate laser light source light having one or more wavelengths in the UV, visible, or infrared, especially having a wavelength selected from the spectral wavelength range of 200-2000 nm, such as 300-1500 nm. The term "laser" especially refers to a device that emits light through a process of optical amplification based on the stimulated emission of electromagnetic radiation.

Especially, in embodiments the term "laser" may refer to a solid-state laser. In specific embodiments, the terms "laser" or "laser light source", or similar terms, refer to a laser diode (or diode laser).

Hence, in embodiments the light source comprises a laser light source. In embodiments, the terms "laser" or "solid state laser" or "solid state material laser" may refer to one or more of cerium doped lithium strontium (or calcium) aluminum fluoride (Ce:LiSAF, Ce:LiCAF), chromium doped chrysoberyl (alexandrite) laser, chromium ZnSe (Cr:ZnSe) laser, divalent samarium doped calcium fluoride (Sm:CaF₂) laser, Er:YAG laser, erbium doped and erbium-ytterbium codoped glass lasers, F-Center laser, holmium YAG (Ho:YAG) laser, Nd:YAG laser, NdCrYAG laser, neodymium doped yttrium calcium oxoborate Nd:YCa₄O(BO₃)₃ or Nd:YCOB, neodymium doped yttrium orthovanadate (Nd:YVO₄) laser, neodymium glass (Nd:glass) laser, neodymium YLF (Nd:YLF) solid-state laser, promethium 147 doped phosphate glass (147Pm³⁺:glass) solid-state laser, ruby laser (Al₂O₃:Cr³⁺), thulium YAG (Tm:YAG) laser, titanium sapphire (Ti:sapphire; Al₂O₃:Ti³⁺) laser, trivalent uranium doped calcium fluoride (U:CaF₂) solid-state laser, Ytterbium doped glass laser (rod, plate/chip, and fiber), Ytterbium YAG (Yb:YAG) laser, Yb₂O₃ (glass or ceramics) laser, etc.

For instance, including second and third harmonic generation embodiments, the light source may comprise one or more of an F center laser, an yttrium orthovanadate (Nd:YVO₄) laser, a promethium 147 doped phosphate glass (147Pm³⁺:glass), and a titanium sapphire (Ti:sapphire; Al₂O₃:Ti³⁺) laser. For instance, considering second and third harmonic generation, such light sources may be used to generated blue light.

In embodiments, the terms "laser" or "solid state laser" or "solid state material laser" may refer to one or more of a semiconductor laser diodes, such as GaN, InGaN, AlGaInP, AlGaAs, InGaAsP, lead salt, vertical cavity surface emitting laser (VCSEL), quantum cascade laser, hybrid silicon laser, etc.

A laser may be combined with an upconverter in order to arrive at shorter (laser) wavelengths. For instance, with some (trivalent) rare earth ions upconversion may be obtained or with non-linear crystals upconversion can be obtained. Alternatively, a laser can be combined with a downconverter, such as a dye laser, to arrive at longer (laser) wavelengths.

As can be derived from the below, the term "laser light source" may also refer to a plurality of (different or identical) laser light sources. In specific embodiments, the term "laser light source" may refer to a plurality N of (identical) laser light sources. In embodiments, N=2, or more. In specific embodiments, N may be at least 5, such as especially at least 8. In this way, a higher brightness may be obtained. In embodiments, laser light sources may be arranged in a laser bank (see also above). The laser bank may in embodiments comprise heat sinking and/or optics e.g. a lens to collimate the laser light. Hence, in embodiments lasers in a laser bank may share the same optics.

The laser light source is configured to generate laser light source light (or "laser light"). The light source light may essentially consist of the laser light source light. The light source light may also comprise laser light source light of two or more (different or identical) laser light sources. For instance, the laser light source light of two or more (different or identical) laser light sources may be coupled into a light guide, to provide a single beam of light comprising the laser light source light of the two or more (different or identical) laser light sources. In specific embodiments, the light source light is thus especially collimated light source light. In yet further embodiments, the light source light is especially (collimated) laser light source light.

The laser light source light may in embodiments comprise one or more bands, having band widths as known for lasers. In specific embodiments, the band(s) may be relatively sharp line(s), such as having full width half maximum (FWHM) in the range of less than 20 nm at RT, such as equal to or less than 10 nm. Hence, the light source light has a spectral power distribution (intensity on an energy scale as function of the wavelength) which may comprise one or more (narrow) bands.

The beams (of light source light) may be focused or collimated beams of (laser) light source light. The term "focused" may especially refer to converging to a small spot. This small spot may be at the discrete converter region, or (slightly) upstream thereof or (slightly) downstream thereof. Especially, focusing and/or collimation may be such that the cross-sectional shape (perpendicular to the optical axis) of the beam at the discrete converter region (at the side face) is essentially not larger than the cross-section shape (perpendicular to the optical axis) of the discrete converter region (where the light source light irradiates the discrete converter region). Focusing may be executed with one or more optics, like (focusing) lenses. Especially, two lenses may be applied to focus the laser light source light. Collimation may be executed with one or more (other) optics, like collimation elements, such as lenses and/or parabolic mirrors. In embodiments, the beam of (laser) light source light may be relatively highly collimated, such as in embodiments ≤2° (FWHM), more especially ≤1° (FWHM), most especially ≤0.5° (FWHM). Hence, ≤2° (FWHM) may be considered (highly) collimated light source light. Optics may be used to provide (high) collimation (see also above).

The term "solid state material laser", and similar terms, may refer to a solid state laser like based on a crystalline or glass body dopes with ions, like transition metal ions and/or lanthanide ions, to a fiber laser, to a photonic crystal laser, to a semiconductor laser, such as e.g. a vertical cavity surface-emitting laser (VCSEL), etc.

The term "solid state light source", and similar terms, may especially refer to semiconductor light sources, such as a light emitting diode (LED), a diode laser, or a superluminescent diode.

Instead of the term "solid state light source" also the term "semiconductor-based light source" may be applied. Hence, the term "semiconductor-based light source" may e.g. refer to one or more of a light emitting diode (LED), a diode laser, and a superluminescent diode.

Hence, the light generating device may comprise one or more of a light emitting diode (LED), a diode laser, and a superluminescent diode.

As indicated above, especially the n1 light generating devices may comprise one or more laser devices, more especially diode lasers. This may provide a relatively high intensity and may also allow the desired collimation (in one direction). Hence, the device light generated by the n1 light generating devices may comprise laser light. Especially, in embodiments the device light of the n1 light generating devices is laser light. The one or more laser devices, such as especially n laser devices, may be laser diodes. However, other embodiments may also be possible (see above). Further, other devices may also be possible, like LEDs.

Especially, the device light is UV radiation. In embodiments, the n1 light generating devices may be configured to generate device light having a wavelength selected from the wavelength range of 100 to 380 nm. As indicated above, such device light may have a disinfection function as it may be detrimental for bacteria and/or viruses. Hence, the system may also be indicated as "disinfection system" or "disinfection light generating system".

Further, optics may be used to create the desired beam shape (see also above and below). To this end the light generating system may further comprise optics. In general, the term "optics" may especially refer to (one or more) optical elements. Hence, the terms "optics" and "optical elements" may refer to the same items. The optics may include one or more or mirrors, reflectors, collimators, lenses, prisms, diffusers, phase plates, polarizers, diffractive elements, gratings, dichroics, arrays of one or more of the afore-mentioned, etc. Alternatively or additionally, the term "optics" may refer to a holographic element or a mixing rod. In embodiments, the optics may include one or more of beam expander optics and zoom lens optics. See further above for examples of optics. In embodiments, the optics may comprise an integrator, like a "Koehler integrator" (or "Köhler integrator").

Herein, especially the n1 light generating devices and the optics may be configured to generate k1 beams of (beam-shaped) device light. Especially, the beam-shaped device light may have a cross-section which is relatively broad in one direction and relatively narrow in an opposite direction. This may allow a narrow beam of light that may propagate along a ceiling and stay close to the ceiling. The system light may comprise at least one beam of device light.

When k1=1, the system light may thus comprise this beam of light (in an operational mode of the system).

When k1 is at least two, the system light may comprise in an operational mode of the system all k1 beams of light. However, in other embodiments, in an operational mode the system light may comprise one or more of the k1 beams of light.

In embodiments, each beam has a first beam angle (θ1) and perpendicular thereto a second beam angle (θ2). Especially, the first beam angle (θ1) and the second beam angle (θ2) may be defined by a full width half maximum of the beam. As known in the art, the maxima may especially be the maxima related to the power (Watts), such as based on the irradiance.

In specific embodiments, the first beam angle (θ1) and the second beam angle (θ2) differ, especially in that one is larger than a predetermined value and the other is smaller than the predetermined value. Especially, in specific embodiments the predetermined value may be selected from the range of 1.5-5°, more especially selected from the range of 2-5°. For instance, one beam angle may be 2°, or smaller and the other beam angle may be 5°, or larger, such as at least 10°. Especially, θ1/θ2 may be larger than 2 or smaller than 0.5. The phrase, predetermined value may be selected from the range of 1.5-5°, and similar phrases, may thus also include embodiments wherein one of the beam angles is smaller than 1.5° and a beam angle perpendicular thereto is at least 6°, as there is a predetermined value selected from the range of 1.5-5° to which one of the beam angles is smaller and the other of the beam angles is larger. In specific embodiments, one beam angle may be at least 10°, such as at least 15°. Hence, with the present invention beams may be provided that have a narrow elongated cross-section; i.e. a relatively high aspect ratio, such as larger than 2 or smaller than 0.5, or even larger than 4 or smaller than 0.25.

In embodiments, one of the first beam angle (θ1) and the second beam angle (θ2) may be selected from the range of at least 4°, more especially at least 5°, and the other one of the first beam angle (θ1) and the second beam angle (θ2) may be selected from the range of at maximum 3°, more especially at maximum 2.5°, such as 2° or smaller.

In specific embodiments, one of the first beam angle (θ1) and the second beam angle (θ2) may be selected from the range of at least 10° and the other one of the first beam angle (θ1) and the second beam angle (θ2) may be selected from the range of at maximum 1.5°. Note that when k1 is at least two, the first beam angles of different beams may all be the same, or two or more may be the same and one or more may differ therefrom. Likewise, when k1 is at least two, the second beam angles of different beams may all be the same, or two or more may be the same and one or more may differ therefrom. Hence, the first beam angles and the second beam angles may individually be chosen for different beams.

In embodiments, n1≥1. In embodiments, n1≥2. Further, in embodiments k1≥1. In embodiments, k1≥2. Especially, in embodiments k1=n1.

As there may be multiple beams, it may be desirable to provide this in a way that they can propagate relatively horizontally, such as skimming a ceiling. Hence, in embodiments, the optical axes of the beams may be configured parallel. Alternatively or additionally, the optical axis may be configured in a single plane. The latter may include embodiments such as parallel beams or radially oriented beams.

In a specific embodiment, a single radial beam may be provided, such as on the basis of a single light generating device. In such embodiments, one of θ1 and θ2 may thus be 360°.

In other embodiments, a plurality of beams may provide a substantially radial beam. For instance, k1 beams may be provided, with second beam angles of one of θ1 and θ2 in the range of about 360°/k1. In such embodiments, a plurality of k1 beams, wherein k1 is at least 2, may provide a radial beam (or radially configured beams).

In yet other embodiments, k2 sets of beams may be provided, wherein within each set the optical axes of the beams may be parallel. For instance, k2 may be 2, 4, or 6, providing sets of beams where the optical axes of adjacent sets have mutual angles 360°/k2. Hence, optical axes may be considered to be configured antiparallel.

However, deviations thereof may also be possible. Hence, in embodiments each k1 beam may have an optical axis (Ok), wherein the optical axes (Ok) are configured in a space (such as in embodiments aligned) between two virtual cones (having parallel cone axes, especially coinciding cone axes), pointing with the apexes to each other, and having apex angles individually selected from the range of 160-180°, more especially 170-180°. In specific embodiments, each beam may be configured to provide light source radiation in a negative space between two virtual (truncated) cones (or double (truncated) cone), connected at the (virtual) apex, and having apex angles individually selected from the range of 330-360 deg², more especially 340-360 deg². Note that a single beam may be a radial beam. Further, a single beam may in general comply anyhow with the above condition. The above condition may especially be relevant when k1 is larger than 1.

As the light generating system may not be a point light source, instead of cones, truncated cones may be applied. Therefore, (more in general) in embodiments each k1 beam may have an optical axis (Ok), wherein the optical axes (Ok) are configured in a space (such as in embodiments aligned) between two virtual truncated cones (having parallel cone axes, especially coinciding cone axes), pointing with the (virtual) apexes to each other, and having apex angles individually selected from the range of 160-180°, more especially 170-180°. The truncated cones may especially be right cones. Such truncated cones may define a cylindrical space, between the two smaller bases (where the cones are truncated). Such cylindrical space may in specific embodiments be a (virtual) cylinder, more especially the smallest (virtual) cylinder, enclosing a light exit window of the system.

Yet even more in general, especially when k1 is at least 2, the optical axes may in a radial plane have essentially any angle, whereas in a plane perpendicular to such radial plane, the angles with that plane may be relatively small. Referring to an embodiment, just for explanation, wherein a plurality of beams is provided substantially parallel to a horizontal ceiling, θ1 may e.g. be the beam angle in a plane parallel to the ceiling and θ2 may be the beam angle in a plane perpendicular to the ceiling. In such embodiments, mutual angles between optical axes projected on a plane parallel to the ceiling may essentially have any value, whereas mutual angles between optical axes projected on a plane perpendicular to the ceiling may be relatively small, such as not more than 15° as largest mutual angle, like not more than 10°. In specific embodiments mutual angles between optical axes projected on the plane perpendicular to the ceiling may not more than 5° as largest mutual angle. Therefore, in embodiments mutual angles between optical axis projected on a first plane may have essentially any value, whereas mutual angles between the optical axes projected on a second plane may, perpendicular to the first plane, may be not more than 15° for the largest mutual angle, like not more than 10°.

In specific embodiments, especially wherein k1 ≥2, each beam may have an optical axis (Ok), wherein the (k1) optical axes (Ok) have third angles (θ3) relative to a virtual plane, wherein the third angles (θ3) are selected from the range of 0-15°. For instance, referring to the above example, the virtual plane may be parallel to the horizontal ceiling. For different beams, the third angles may vary.

Especially, the optical axis may be defined as an imaginary line that defines the path along which light propagates through a system starting from the light generating element, here especially the light source. Especially, the optical axis may coincide with the direction of the light with the highest radiant flux.

Further, the light generating system may be configured to generate system light comprising at least one beam of device light. Dependent upon whether two or more of the beams may be controlled individually, or whether different sets of beams may be controlled individually, the system light may comprise one or more beams of device light. For course, when k1=1, the system light may comprise a single beam of device light.

The UV radiation may be provided as such or may be the result of an upconversion process. Hence, in embodiments a visible laser or an infrared laser may be used in combination with one or more of a frequency doubling material or an upconversion luminescent materials, thereby providing UV (laser) light. Therefore, in embodiments the n1 light generating devices may comprise one or more first light sources configured to generate infrared or visible first light source light and an upconverter material, wherein the one or more first light sources and the upconverter material are configured to generate the device light having the wavelength in the wavelength range of 100 to 380 nm. Frequency doubling may also refer to triplicating a frequency or multiplications of one or more of doubling (now referring to 2x only) and triplicating. In specific embodiments, the one or more first light sources may comprise solid state light sources, such as especially laser diodes.

However, in other embodiments a direct UV emitting light source, more especially a (direct) UV emitting laser may be applied, like a UV diode laser. Therefore, in embodiments the n1 light generating devices may comprise one or more second light sources configured to generate second light source light having a wavelength in the wavelength range of 100 to 380 nm, wherein the device light may comprise the second light source light. In specific embodiments, the one or more second light sources may comprise solid state light sources, such as especially laser diodes.

With the present invention, in embodiments it may also be possible to provide light having different spectral power distributions. For instance, a light generating device may be configured to generate UV radiation having intensities at smaller wavelengths than another light generating device. As can be derived from above table, this may add to the versatile applicability of the light generating system. For instance, in this way it may be possible to disinfect a broader spectrum of bacteria and/or a broader spectrum of viruses.

Hence, in embodiments two or more light generating devices may be configured to generate device light having different centroid wavelengths, having a centroid wavelength difference of at least 10 nm, more especially at least 15 nm. In specific embodiments, two or more light generating devices may be configured to generate device light having different centroid wavelengths differing least 20 nm, such as at least 25 nm, like in specific embodiments at least 30 nm.

The term "centroid wavelength", also indicated as λc, is known in the art, and refers to the wavelength value where half of the light energy is at shorter and half the energy is at longer wavelengths; the value is stated in nanometers (nm). It is the wavelength that divides the integral of a spectral power distribution into two equal parts as expressed by the formula λc = Σ λ*I(1) / (Σ I(λ)), where the summation is over the wavelength range of interest, and I(λ) is the spectral energy density (i.e. the integration of the product of the wavelength and the intensity over the emission band normalized to the integrated intensity). The centroid wavelength may e.g. be determined at operation conditions.

In specific embodiments, two or more light generating devices may be configured to generate device light in different wavelength ranges selected from a UV-A wavelength range, a UV-B wavelength range, a Near UV-C wavelength range, and a Far UV-C wavelength range.

In embodiments, k1 may be at least 2. When there are two or more beams, it may be desirable that they propagate substantially parallel. For instance, in embodiments two or more light generating devices are configured such that the k1 beams have parallel first beam angles (θ1) and/or parallel second beam angles (θ2). Hence, the optical axes may essentially be parallel. Alternatively, the optical axis may essentially be in a plane, and may e.g. be configured under angles relative to each other. For instance, in such way it may be possible to provide a substantially radial beam of device light, composed of a plurality of beams of light. Yet alternatively, see also below, it may be possible to provide a single beam of light having a radial shape.

In embodiments, the system may comprise a light exit, like an end window or an (other) optical element (of material transmissive for the device light, e.g. quartz), or an opening, from which the system light may escape to the external of the system. The system may comprise a housing, comprising such light exit. The housing may at least partly enclose one or more light generating devices and one or more (other) optical elements.

In specific embodiments, the light generating system may comprise a light exit, via which the system light may escape from the system, where at the light exit, beam widths (W2) of the k1 beams defined by the first beam angles (θ1) may be at least 1 cm. The beam(s) leaving the light exit may be sufficiently broad such that safety may be high.

The beams may be provided in an array, such as a rectangular array. Hence, in embodiments, wherein k1≥4, more especially wherein k1≥5, the light generating system is configured to generate an N*M array of the beams of device light, wherein N≥2 and wherein M≥2. In embodiments, N may be larger than M. In embodiments, the larger number of N may provide arrays of beams parallel to the largest of the first beam angle and the second beam angle.

Especially, the beams of light escaping from the system may provide a broad beam in one direction (such as in application parallel to a ceiling), but relatively narrow in an opposite direction (such as in application perpendicular to a ceiling). It may also be desirable when there are a plurality of beams, a total height defined by the beams may relatively small, whereas the width may be relatively large. Two or more beams may provide a largest width W3 in the exit and a largest height H3 in the exit. The largest width and the largest height may be (also) be provided by the full width half maxima (of different beams). Especially W3/H3≥2, such as W3/H3≥5.

Examples of optics have also been provided above. In specific embodiments, the optics may at least comprise collimating optics. Further, e.g. diffractive, or refractive optics, or a combination thereof, may be used to (further) shape the beam of device light.

In specific embodiments, the optics may comprise (i) first optics, wherein the first optics comprise lenses configured to collimate the device light, and (ii) second optics, wherein the second optics comprise one or more of diffractive optics and refractive optics, configured to impose the shape of the beams, especially with a higher aspect ratio than upstream of the second optics. For instance, such optics may be used in combination with laser devices comprising laser diodes.

The terms "upstream" and "downstream" relate to an arrangement of items or features relative to the propagation of the light from a light generating means (here the especially the light source), wherein relative to a first position within a beam of light from the light generating means, a second position in the beam of light closer to the light generating means is "upstream", and a third position within the beam of light further away from the light generating means is "downstream".

With diffractive or refractive optics, it may be possible to (further) create a substantially line-shaped beam of (laser) device light. However, with diffractive or refractive optics, it may also be possible to (further) create a circular shaped beam of (laser) device light.

A circular shaped beam of (laser) device light may, e.g. in combination with a conical specular reflector, be converted in a radial beam of (laser) device light. The n1 light generating devices and optics are configured to generate a radially configured beam of (beam-shaped) device light. In a specific embodiments n1=k1=1 (while the beam is a radial beam). The radial beam may provide device light in substantially any direction (within a (virtual) plane).

When there are controllable elements and/or when there are a plurality of individually controllable elements, e.g. a control system may be used to control the controllable elements. For instance, a control system may control l1 sets of light generating devices, wherein each set comprises at least one light generating device, and wherein 11 is at least 2.

The term "controlling" and similar terms especially refer at least to determining the behavior or supervising the running of an element. Hence, herein "controlling" and similar terms may e.g. refer to imposing behavior to the element (determining the behavior or supervising the running of an element), etc., such as e.g. measuring, displaying, actuating, opening, shifting, changing temperature, etc. Beyond that, the term "controlling" and similar terms may additionally include monitoring. Hence, the term "controlling" and similar terms may include imposing behavior on an element and also imposing behavior on an element and monitoring the element. The controlling of the element can be done with a control system, which may also be indicated as "controller". The control system and the element may thus at least temporarily, or permanently, functionally be coupled. The element may comprise the control system. In embodiments, the control system and element may not be physically coupled. Control can be done via wired and/or wireless control. The term "control system" may also refer to a plurality of different control systems, which especially are functionally coupled, and of which e.g. one control system may be a master control system and one or more others may be slave control systems. A control system may comprise or may be functionally coupled to a user interface.

The control system may also be configured to receive and execute instructions from a remote control. In embodiments, the control system may be controlled via an App on a device, such as a portable device, like a Smartphone or I-phone, a tablet, etc. The device is thus not necessarily coupled to the lighting system, but may be (temporarily) functionally coupled to the lighting system.

Hence, in embodiments the control system may (also) be configured to be controlled by an App on a remote device. In such embodiments the control system of the lighting system may be a slave control system or control in a slave mode. For instance, the lighting system may be identifiable with a code, especially a unique code for the respective lighting system. The control system of the lighting system may be configured to be controlled by an external control system which has access to the lighting system on the basis of knowledge (input by a user interface of with an optical sensor (e.g. QR code reader) of the (unique) code. The lighting system may also comprise means for communicating with other systems or devices, such as on the basis of Bluetooth, WIFI, LiFi, ZigBee, BLE or WiMAX, or another wireless technology.

The system, or apparatus, or device may execute an action in a "mode" or "operation mode" or "mode of operation" or "operational mode". The term "operational mode may also be indicated as "controlling mode". Likewise, in a method an action or stage, or step may be executed in a "mode" or "operation mode" or "mode of operation" or "operational mode". This does not exclude that the system, or apparatus, or device may also be adapted for providing another controlling mode, or a plurality of other controlling modes. Likewise, this may not exclude that before executing the mode and/or after executing the mode one or more other modes may be executed.

However, in embodiments a control system may be available, that is adapted to provide at least the controlling mode. Would other modes be available, the choice of such modes may especially be executed via a user interface, though other options, like executing a mode in dependence of a sensor signal or a (time) scheme, may also be possible. The operation mode may in embodiments also refer to a system, or apparatus, or device, that can only operate in a single operation mode (i.e. "on", without further tunability).

Hence, in embodiments, the control system may control in dependence of one or more of an input signal of a user interface, a sensor signal (of a sensor), and a timer. The term "timer" may refer to a clock and/or a predetermined time scheme.

Hence, in embodiment the system may further comprise a control system. Especially, the control system may be configured to control the n1 light generating devices.

In embodiments, the control system may control the system light in dependence of a sensor. In embodiments, the sensor may be selected from the group comprising a movement sensor, a presence sensor, a distance sensor, an ion sensor, a gas sensor, a volatile organic compound sensor, a pathogen sensor, an airflow sensor, a sound sensor, a temperature sensor, and a humidity sensor. A movement sensor may be used to sense people. A movement sensor may also be used to sense the number of people. A movement sensor may also be used to sense an activity level of the people (e.g. occupied or non-occupied working cubicle or fitness room). A presence sensor may be used to sense people. A presence sensor may also be used to sense the number of people. A presence sensor may also be used to sense an activity level of the people (e.g. occupied or non-occupied working cubicle or fitness room). A distance sensor may be used to sense one or more dimensions of a space for which the ionizer device is used. A distance sensor may also be used to sense distances between people. The ion sensor may comprise a positive ion sensor. Additionally or alternatively, the ion sensor may comprise a negative ion sensor. The ion sensor may be used to sense the effect of the ionizer device (the more ions, the better the air treatment may be). A gas sensor may be used to sense gas one or more gas components. The gas sensor may be used to sense whether ventilation is sufficient or insufficient. The gas sensor may e.g. (thus) also be used to sense the number of people and/or an activity level of the people. A volatile organic compound (VOG) sensor may be used to sense one or more volatile organic compounds. The VOG sensor may be used to sense whether ventilation is sufficient or insufficient. The VOG sensor may e.g. (thus) also be used to sense the number of people and/or an activity level of the people. The pathogen sensor may comprise a sensor for one or more of bacteria, viruses, and spores. The pathogen sensor may be used to sense whether ventilation is sufficient or insufficient. The pathogen sensor may e.g. (thus) also be used to sense the number of people and/or an activity level of the people. An airflow sensor may be used to sense an airflow. The airflow sensor may be used to sense whether ventilation is sufficient or insufficient. The airflow sensor may e.g. (thus) also be used to sense the number of people and/or an activity level of the people. A sound sensor may be used to sense sound. The sound sensor may be used to sense whether ventilation is sufficient or insufficient. The sound sensor may e.g. (thus) also be used to sense the number of people and/or an activity level of the people. The temperature sensor may be used to sense temperature. On the basis thereon, it may be determined whether pathogens may be more detrimental or less detrimental. The humidity sensor may be used to sense (air) humidity. On the basis thereon, it may be determined whether pathogens may be more detrimental or less detrimental (as there seems to be a relation between humidity and transferability of e.g. airborne pathogens).

When controllable optics would be available, the control system may be configured to control the controllable optics (as well). Hence, in embodiments the control system may be configured to control one or more of (i) the n1 light generating devices, and (ii) optional controllable optics, wherein the optional controllable optics are configured to control a propagation direction of at least one of the k1 beams.

In embodiments, controllable optics may be applied, like e.g. a lens having a controllable focal point or a mirror which may be rotated, etc. By controlling such optics, beam shape and/or direction may be controlled. Hence, in embodiments the control system is configured to change one or more of a direction and a shape of the at least one beam by controlling the controllable optics. The term "controllable" may especially indicate that elements may be controlled electrically, like e.g. via actuators or by applying a voltage to a material.

In specific embodiments, it may be desirable that it is visible that the UV radiation is provided. For instance, visible light may be admixed to the UV radiation and/or a kind of visible light edge may be provided to the UV radiation. Hence, in embodiments the system may also comprise a source of visible light. Such visible light may be provided by converting a (small) part of the UV radiation and/or by a separate light generating device (indicated as "second light generating device"). Here below, especially the latter embodiment is described, as the latter embodiment may allow control of the visible light essentially independent of the UV radiation. The visible light may be white light or colored light. In specific embodiments, the visible light may be violet light.

The terms "violet light" or "violet emission" especially relates to light having a wavelength in the range of about 380-440 nm. The terms "blue light" or "blue emission" especially relates to light having a wavelength in the range of about 440-495 nm (including some violet and cyan hues). The terms "green light" or "green emission" especially relate to light having a wavelength in the range of about 495-570 nm. The terms "yellow light" or "yellow emission" especially relate to light having a wavelength in the range of about 570-590 nm. The terms "orange light" or "orange emission" especially relate to light having a wavelength in the range of about 590-620 nm. The terms "red light" or "red emission" especially relate to light having a wavelength in the range of about 620-780 nm. The term "pink light" or "pink emission" refers to light having a blue and a red component. The term "cyan" may refer to one or more wavelengths selected from the range of about 490-520 nm. The term "amber" may refer to one or more wavelengths selected from the range of about 585-605 nm, such as about 590-600 nm. The phrase "light having one or more wavelengths in a wavelength range" and similar phrases may especially indicate that the indicated light (or radiation) has a spectral power distribution with at least intensity or intensities at these one or more wavelengths in the indicate wavelength range. For instance, a blue emitting solid state light source will have a spectral power distribution with intensities at one or more wavelengths in the 440-495 nm wavelength range.

Especially, the second device light of the second light generating device may in embodiments have substantially the same direction and beam shape as the (first) device light of (first) light generating device(s). Hence, the beam angles may essentially be the same and the optical axis may substantially be parallel to at least one of the optical axes of the device light. Hence, essentially all embodiments described above in relation to the light generating devices and optics, may also apply for the second light generating device (and its optional optics).

Therefore, in embodiments the light generating system may further comprise a second light generating device, wherein the second light generating device is configured to generate visible second device light, more especially having a wavelength selected from the 380-440 nm wavelength range; wherein the second light generating device and the optics are configured to generate a second beam of second device light, wherein the second beam has a first beam angle (θ21) and perpendicular thereto a second beam angle (θ22), wherein the first beam angle (θ21) and the second beam angle (θ22) are defined by a full width half maximum of the second beam, wherein one of the first beam angle (θ21) and the second beam angle (θ22) is selected from the range of at least 5° and the other one of the first beam angle (θ21) and the second beam angle (θ22) is selected from the range of at maximum 2°. In specific embodiments, the second light generating device may comprise a laser diode.

Hence, there may be one or more light generating devices configured to provide the UV radiation and one or more second light generating devices configured to generate the visible second device light, especially violet second device light. The optical axis of the second device light may in embodiments essentially coincide with at least one of the optical axis of the k1 beams. Would the device light be provided as a radial beam, then also the second device light may be provided as radial beam. Especially, the beam of second device light may at least partly overlap with one or more beams of device light.

It may also be possible to combine beams of light. Especially, when beams of light have different spectral power distribution, beams of light may be combined. For instance, a reflective polarizer and/or a UV dichroic mirror may be used to combine beams of light. Other beam combines may be applied as well.

Therefore, in specific embodiments the optics may (further) comprises one or more of a reflective polarizer and UV dichroic mirror configured to combine beams (105,106) of device light (101,121) having different spectral power distributions.

The light generating system may be part of or may be applied in e.g. office lighting systems, household application systems, shop lighting systems, home lighting systems, accent lighting systems, spot lighting systems, theater lighting systems, fiber-optics application systems, projection systems, self-lit display systems, pixelated display systems, segmented display systems, warning sign systems, medical lighting application systems, indicator sign systems, decorative lighting systems, portable systems, automotive applications, (outdoor) road lighting systems, urban lighting systems, green house lighting systems, horticulture lighting, digital projection, or LCD backlighting. The light generating system (or luminaire) may be part of or may be applied in e.g. optical communication systems or disinfection systems.

In yet a further aspect, the invention also provides a lamp or a luminaire comprising the light generating system as defined herein. The luminaire may further comprise a housing, optical elements, louvres, etc. etc... The lamp or luminaire may further comprise a housing enclosing the light generating system. The lamp or luminaire may comprise a light window in the housing or a housing opening, through which the system light may escape from the housing. In yet a further aspect, the invention also provides a projection device comprising the light generating system as defined herein. Especially, a projection device or "projector" or "image projector" may be an optical device that projects an image (or moving images) onto a surface, such as e.g. a projection screen. The projection device may include one or more light generating systems such as described herein. Hence, in an aspect the invention also provides a light generating device selected from the group of a lamp, a luminaire, a projector device, a disinfection device, a photochemical reactor, and an optical wireless communication device, comprising the light generating system as defined herein. The light generating device may comprise a housing or a carrier, configured to house or support, one or more elements of the light generating system. For instance, in embodiments the light generating device may comprise a housing or a carrier, configured to house or support one or more of the n1 light generating devices and the optics. The lighting device may be a suspension lighting device. Such device may be suspended from a ceiling.

In yet a further aspect, the invention also provides a method for treating a gas or a surface in a space, wherein the space is external from the light generating system as described herein, the method comprising providing system light (in the space) to the gas or the surface with the light generating system. As indicated above, the system light may especially comprise UV radiation.

In embodiments, the method may comprise: providing system light in the space such that undisturbed beams are only available in a space part h1 meters above a floor in the space, wherein h1 is selected from the range of at least 220 cm; wherein the k1 beams have optical axes (Ok), wherein the optical axes (Ok) have an angle (θ4) with a horizontal selected from the range of 0-15°. Hence, relative to a horizontal, the optical axis may have an angle of at maximum 15°, with the horizontal. This may thus include the range of +15° to - 15°. For instance, the optical axes (Ok) may have an angle (θ4) with a horizontal selected from the range of 0-10°, such as 0-10°.

In embodiments, the one or more beams may skim along a horizontal ceiling. In embodiments, the system may sweep one or more beams.

In specific embodiments, the beams of light may have a small angle relative to the ceiling, such that the device light propagates (slightly) in the direction of the ceiling. The optical axis may be directed at the ceiling, under a relatively small angle. Hence, in embodiments the method may comprise providing system light in the space, wherein the k1 beams have optical axes (Ok), wherein the optical axes (Ok) have an angle (θ4) with a horizontal selected from the range of larger than 0° and equal to or smaller than 15°, and wherein the optical axes (Ok) are directed away from the floor. In this way, the optical axis may be directed to the ceiling.

The terms "light" and "radiation" are herein interchangeably used, unless clear from the context that the term "light" only refers to visible light. The terms "light" and "radiation" may thus refer to UV radiation, visible light, and IR radiation. In specific embodiments, especially for lighting applications, the terms "light" and "radiation" refer to (at least) visible light. The term IR radiation may in specific embodiments refer to near IR radiation (NIR). Therefore, herein also the term "(N)IR" is applied, to refer to in general IR, and in specific embodiments to NIR. Herein, the term "visible light" especially relates to light having a wavelength selected from the range of 380-780 nm. Herein, UV (ultraviolet) may especially refer to a wavelength selected from the range of 190-380 nm, though in specific embodiments other wavelengths may also be possible. Herein, IR (infrared) may especially refer to radiation having a wavelength selected from the range of 780-3000 nm, such as 780-2000 nm, e.g. a wavelength up to about 1500 nm, like a wavelength of at least 900 nm, though in specific embodiments other wavelengths may also be possible. Hence, the term IR may herein refer to one or more of near infrared (NIR (or IR-A)) and short-wavelength infrared (SWIR (or IR-B)), especially NIR.

### BRIEF DESCRIPTION OF THE DRAWINGS

Embodiments of the invention will now be described, by way of example only, with reference to the accompanying schematic drawings in which corresponding reference symbols indicate corresponding parts, and in which:
Figs. 1a-1e schematically depict some embodiments and aspects;
Figs. 2a-2d schematically depicts some further aspects and embodiments; and
Figs. 3a-3b schematically depict some further embodiments and aspects.

The schematic drawings are not necessarily to scale.

### DETAILED DESCRIPTION OF THE EMBODIMENTS

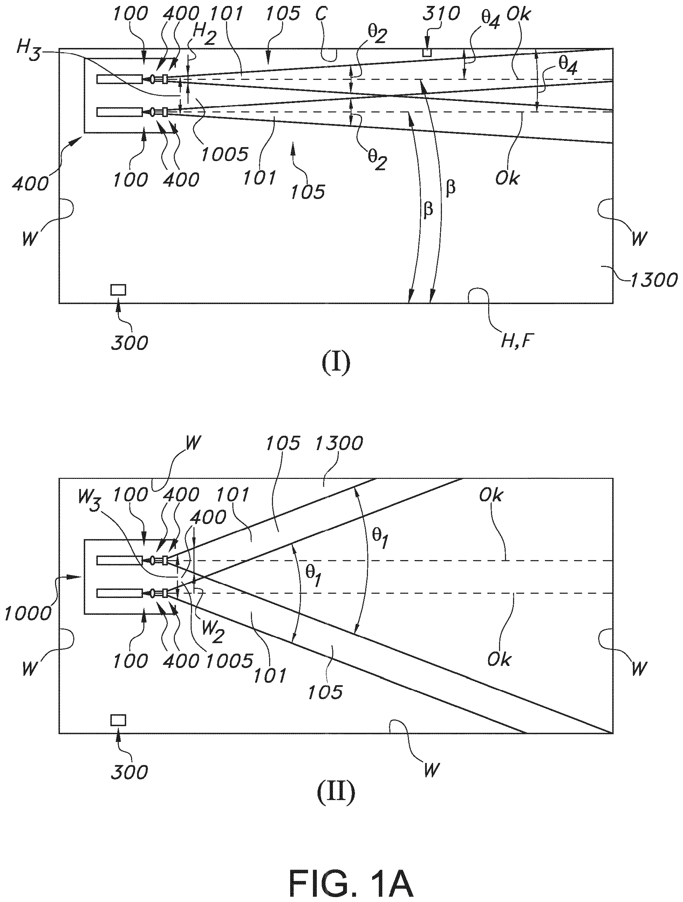

Fig. 1a schematically depicts schematically a space 1300 with a light generating system 1000, with in embodiment I schematically a vertical cross-section and in embodiment II schematically a horizontal cross-section.

The light generating system 1000 comprises n1 light generating devices 100 and optics 400. The n1 light generating devices 100 are configured to generate device light 101 having a wavelength selected from the wavelength range of 100 to 380 nm. Especially, n1≥1. In embodiments, the n1 light generating devices 100 comprise one or more laser devices.

In embodiments, the n1 light generating devices 100 and optics 400 may be configured to generate k1 beams 105 of (beam-shaped) device light 101. Each beam 105 has a first beam angle θ1 and perpendicular thereto a second beam angle θ2. The first beam angle θ1 and the second beam angle θ2 are defined by a full width half maximum of the beam 105. One of the first beam angle θ1 and the second beam angle θ2 may be selected from the range of at least 5°, here the first beam angle θ1, and the other one of the first beam angle θ1 and the second beam angle θ2°, here the second beam angle θ2, may be selected from the range of at maximum 2°. Especially, k1≥1.

The beams 105 may especially be defined by the FWHM (full width half maximum). Here, in Fig. 1a, by way of example k1=2.

Herein, θ1 refers in some of the drawings to the larger beam angle, which may especially be substantially parallel to a ceiling. Further, herein, θ2 refers in some of the drawings to the smaller beam angle, which may especially be in a plane perpendicular to the ceiling.

Each k1 beam 105 may have an optical axis Ok.

The light generating system 1000 may be configured to generate system light 1001 comprising at least one beam 105 of device light 101.

Reference θ4 refers to an angle of the optical axis with a horizontal, such as the ceiling. Reference β refers to an angle of the optical axis with a horizontal, such as the floor. The values may be the same.

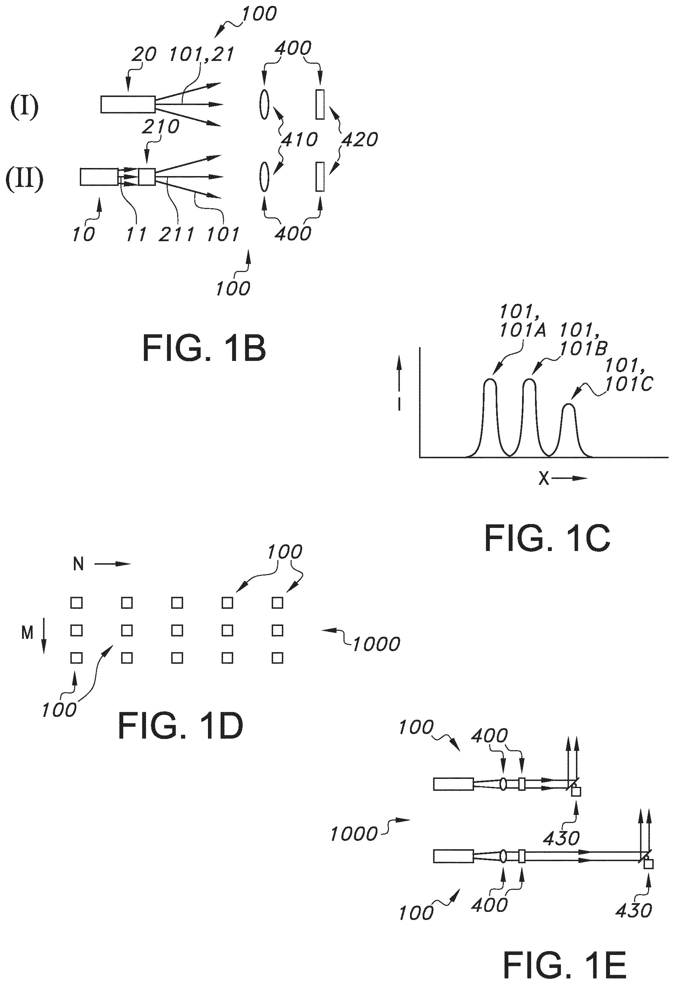

Referring to Fig. 1b, in embodiments, see embodiment II, the n1 light generating devices 100 may comprise one or more first light sources 10 configured to generate infrared or visible first light source light 11 and an upconverter material 210. The one or more first light sources 10 and the upconverter material 210 may be configured to generate the device light 101 having the wavelength in the wavelength range of 100 to 380 nm. Alternatively or additionally, in embodiments, see embodiment I, the n1 light generating devices 100 may comprise one or more second light sources 20 configured to generate second light source light 21 having a wavelength in the wavelength range of 100 to 380 nm. The device light 101 may comprise the second light source light 21.

Referring to Figs. 1a-1c, two or more light generating devices 100 may be configured to generate device light 101 having different centroid wavelengths, having a centroid wavelength difference of at least 15 nm. See the schematically spectral power distributions in Fig. 1c. For instance, in embodiments two or more light generating devices 100 are configured to generate device light 101 in different wavelength ranges selected from a UV-A wavelength range, a UV-B wavelength range, a Near UV-C wavelength range, and a Far UV-C wavelength range.

Referring e.g. to Fig. 1a, in specific embodiments two or more light generating devices 100 may be configured such that the k1 beams 105 have parallel first beam angles θ1 and/or second beam angles θ2.

In specific embodiments, one of the first beam angle θ1 and the second beam angle θ2 may be selected from the range of at least 10° and the other one of the first beam angle θ1 and the second beam angle θ2 may be selected from the range of at maximum 1.5°.

Especially, k1=n1 may apply.

Referring to e.g. Fig. 1a, the light generating system 1000 may comprise a light exit 1005, via which the system light 1001 escapes from the system 1000, where at the light exit 1005, beam widths W2 of the k1 beams 105 defined by the first beam angles θ1 are at least 1 cm. Here, the first beam angle θ1 now refers to the largest beam angle of the beam 105.

Referring to Fig. 1a, embodiment I, the upper horizontal may refer to a horizontal ceiling and the lower horizontal may refer to a floor. The angle θ4 may indicated the angle of an optical axis Ok with the ceiling, which θ4 may differ for different beams 105 (here the optical axis Ok are parallel). By way of example, here the second beam angles θ2 refer to the beam angle in a vertical plane.

Referring to Fig. 1a, embodiment I, the upper horizontal may refer to a wall and the lower horizontal may refer to another wall. As can be seen the beam angles in the horizontal plane can be large. The mutual angle between the optical axis can have essentially any value. Here, the optical axes in this plane are parallel. Note that consequently, by way of example, here the first beam angles θ1 refer to the beam angle in a horizontal plane.

Referring to e.g. Fig. 1d, in specific embodiments k1≥5. Further, in specific embodiments the light generating system 1000 may be configured to generate an N*M array of the beams 105 of device light 101. In embodiments, one or more of N≥2 and M≥2 may apply.

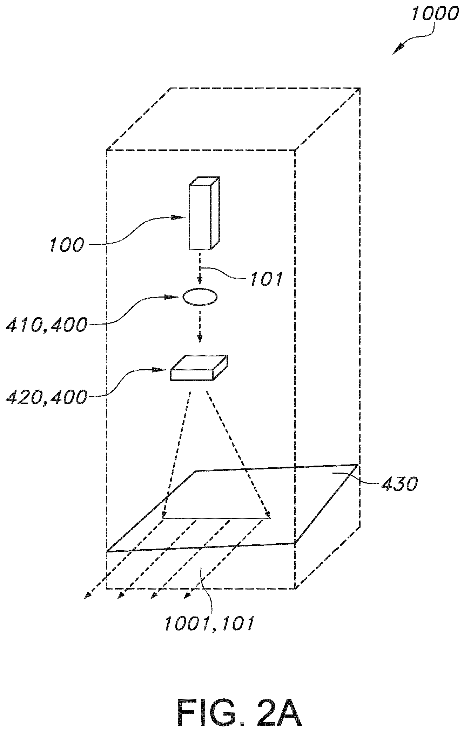

Referring to e.g. Fig. 1b and Figs. 2a-2b, in embodiments the optics 400 may comprise first optics 410. The first optics 410 may comprise lenses configured to collimate the device light 101. In embodiments, the optics 400 may comprise second optics 420. The second optics 420 may comprise one or more of diffractive optics and refractive optics, configured to impose the shape of the beams 105 with a higher aspect ratio than upstream of the second optics 420.

In embodiments, the laser devices comprise laser diodes.

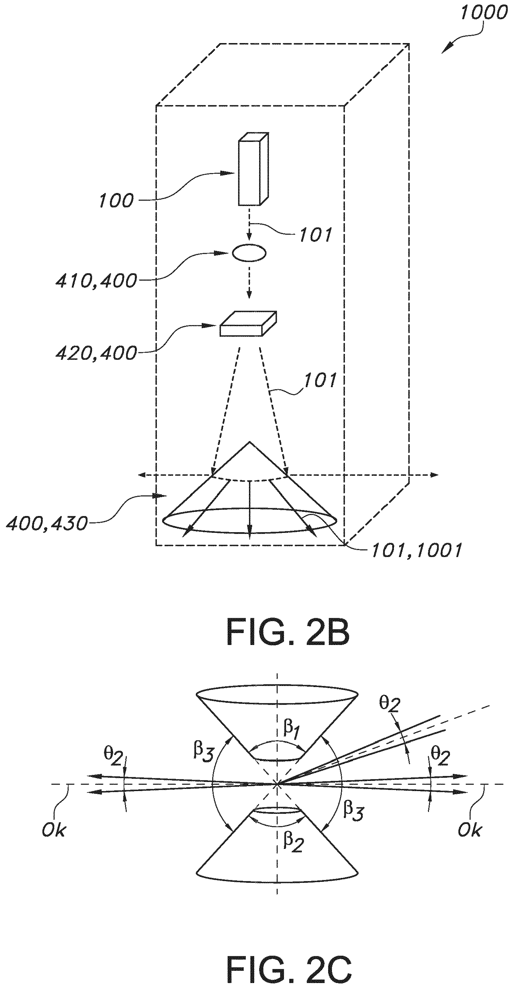

Referring to Fig. 2b, the n1 light generating devices 100 and optics 400 may be configured to generate a radially configured beam 105 of (beam-shaped) device light 101. Reference 430 refers to a conical specular reflector. Second optics 420 may be configured to generate a circular beam of device light 101.

Referring to e.g. Fig. 1a, the light generating system 1000 may further comprising a control system 300. The control system 300 may be configured to control one or more of (i) the n1 light generating devices 100, and (ii) optional controllable optics 430.

In embodiments, also referring to e.g. Fig. 2a, the optional controllable optics 430 may be configured to control a propagation direction of at least one of the k1 beams 105. In embodiments, the control system 300 may be configured to change one or more of a direction and a shape of the at least one beam 105 by controlling the controllable optics 430. Very schematically, an embodiment of controllable optics is also depicted in Fig. 1e. Here, the direction of the beam may e.g. be controlled.

In embodiments, the control system may control the system light in dependence of a sensor. A sensor is indicated with reference 310 in Fig. 1a.

In embodiments, the optical axes Ok may be configured in a space (such as in embodiments aligned) between two virtual (truncated) cones (having parallel cone axes), pointing with the apexes to each other, and having apex angles individually selected from the range of 170-180°, see also Fig. 2c.

The angles β1 and β2 are the cone angles. They may be the same or may be different. Angle β4 is the angle between the two cones. Hence, especially β1+β2+2*β3=360°. Note that β3 may be relatively small, such as at maximum 15°.

In Fig. 2c, even though the optical axes (here dashed lines) may point in different direction, and may have a mutual angle in a vertical plane (as depicted here), the mutual angle between the optical axes may be defined by the two truncated cones. The angles in a horizontal plane may have any (desired) value.

Instead of the terms "vertical plane" and "horizontal plane", also the terms "first plane" and "second plane", respectively, may be used.

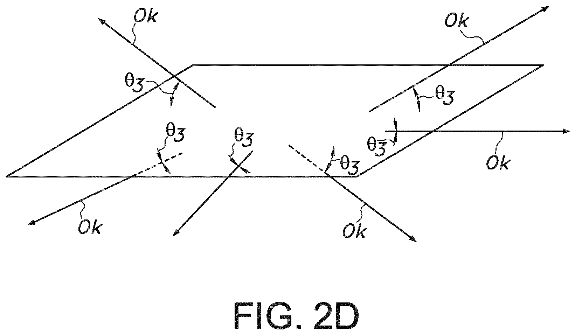

Fig. 2d schematically depicts another way to define the freedom of the optical axis in one plane, and the limited freedom of the optical axis in a direction perpendicular to that plane. As can be seen, especially wherein k1 ≥2, each beam 105 may have an optical axis Ok, wherein the k1 optical axes Ok have k1 third angles θ3 relative to a virtual plane, wherein the third angles θ3 are selected from the range of 0-15°, such as 0-10°. For instance, referring to Fig. 1a, the virtual plane may be parallel to the horizontal ceiling. Would the virtual plane be parallel to the ceiling of Fig. 1a, then θ4=θ3. Here, the optical axis are also provided with a direction (propagation direction of the device light).

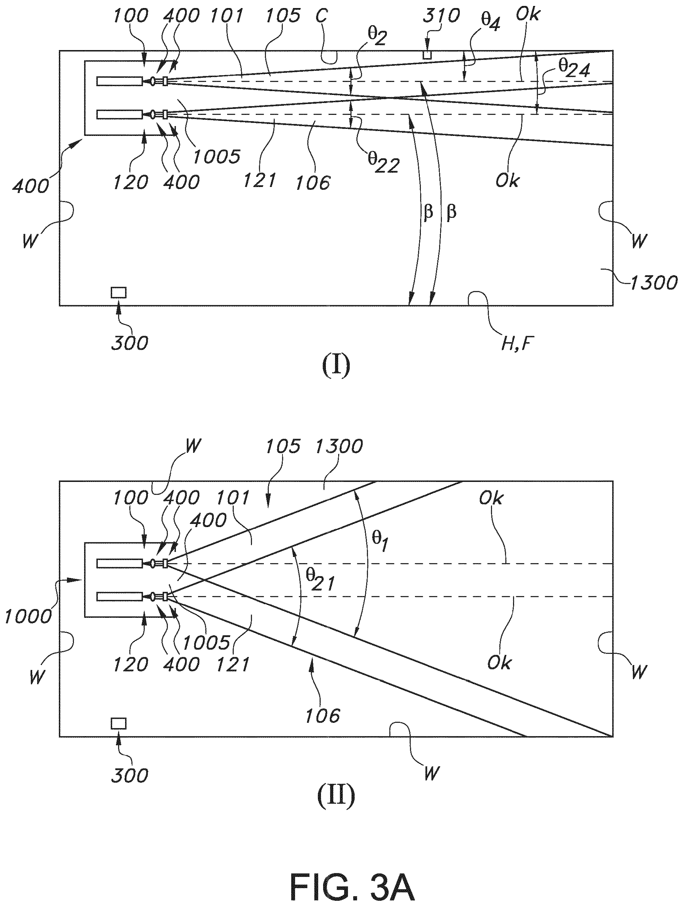

Referring to Fig. 3a, the light generating system 1000 may further comprise a second light generating device 120. The second light generating device 120 may be configured to generate violet second device light 121 having a wavelength selected from the 380-440 nm wavelength range. The second light generating device 120 and the optics 400 may be configured to generate a second beam 106 of second device light. The second beam 106 may have a first beam angle θ21 and perpendicular thereto a second beam angle θ22. The first beam angle θ21 and the second beam angle θ22 are defined by a full width half maximum of the second beam 106. One of the first beam angle θ21 and the second beam angle θ22 may be selected from the range of at least 5° and the other one of the first beam angle θ21 and the second beam angle θ22 may be selected from the range of at maximum 2°. In embodiments, the second light generating device 120 may comprise a laser diode.

The angles θ4 and θ24 are the respective angles of the optical axis with a horizontal, such as the ceiling. Here, like in Fig. 1a, they are about zero degrees (i.e. essentially parallel).

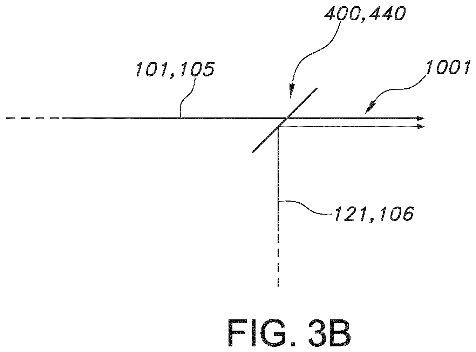

In embodiments, the optics 400 may comprise one or more of a reflective polarizer and UV dichroic mirror configured to combine beams 105,106 of device light 101,121 having different spectral power distributions. An embodiment thereof is schematically depicted in Fig. 3b.

Hence, the invention also provides a method for treating a gas or a surface in a space 1300. The space 1300 may be external from the light generating system 1000. The method may comprise providing system light 1001 (in the space 1300) to the gas, such as air, or the surface with the light generating system 1000. The method may comprise: providing system light 1001 in the space 1300 such that undisturbed beams 105 are only available in a space part h1 meters above a floor in the space 1300; h1 may be selected from the range of at least 220 cm

The k1 beams 105 have optical axes Ok. The optical axes Ok have an angle θ4 with a horizontal selected from the range of 0-15°.

In specific embodiment, the method may comprise providing system light 1001 in the space 1300 such that undisturbed beams 105 are only available in a space part h1 meters above a floor in the space 1300, wherein h1 is selected from the range of at least 220 cm; wherein the k1 beams 105 have optical axes Ok, wherein the optical axes Ok have an angle θ4 with a horizontal selected from the range of larger than 0° and equal to or smaller than 15°, and wherein the optical axes Ok are directed away from the floor.

It is desired to protect yourself and others from the spread of bacteria and viruses such as influenza or against the outbreak of novel viruses like the recent COVID-19. UV light emitted by UV light sources can be used for disinfection. An type of disinfection is upper air disinfection wherein UV light emitted by especially a UV TL tube is collimated by a light absorbing lamellae and emitted only in the upper part of a room. However, the lamellae of the upper air disinfection device may absorb a substantial part of the UV light (i.e. a low efficiency). The spatial light distribution in the vertical direction may be too broad (i.e. safety issues e.g. straylight from the ceiling). The spectral light distribution may be narrow (i.e. some viruses and/or bacteria are better activated at other UV wavelengths). The spatial and/or spectral light distribution may not be controllable.

Amongst others, a UV laser (or LED) light upper air disinfection device is herein proposed. The upper air disinfection device may be configured to, in operation, provide disinfection light. The upper air disinfection device may comprise: a plurality of laser (or LED) light sources providing a plurality of beams of UV light; an array of lenses configured to collimate the beams of UV light into collimated beams UV light; a diffractive or refractive component configured to redirect the collimated beams of UV light into elongated light distributions having a FWHM width in a horizontal plane and a FWHM height in a vertical plane, wherein the FWHM width >5° and FWHM height <2°. In embodiments, the disinfection light may be pixelated laser light and elongated light distributions are parallelly arranged. In embodiments, neighboring elongated light distributions comprises UV light of different wavelengths, particular different UV ranges (UVA/UVB/UVC) in particular more safe UV wavelengths at the bottom of the upper room, thereby providing an improved disinfection performance and safety. In embodiments, non-linear optics (NLO) may be used to convert visible and/or NIR laser light into UV laser light e.g. convert 444 nm and/or 508 nm laser into 222 nm and 254 nm light. This may allow a low cost solution. In embodiments, a width of the elongated beam exiting an exit window of the upper air disinfection device may be at least 1 cm (improved safety). In embodiments, n≥5. In embodiments, the lasers may be arranged in a vertical line configuration. In embodiments, the pixelated laser light may be a matrix of N>5 rows by M>3 columns.

The term "plurality" refers to two or more.

The terms "substantially" or "essentially" herein, and similar terms, will be understood by the person skilled in the art. The terms "substantially" or "essentially" may also include embodiments with "entirely", "completely", "all", etc. Hence, in embodiments the adjective substantially or essentially may also be removed. Where applicable, the term "substantially" or the term "essentially" may also relate to 90% or higher, such as 95% or higher, especially 99% or higher, even more especially 99.5% or higher, including 100%.

The term "comprise" also includes embodiments wherein the term "comprises" means "consists of".

The term "and/or" especially relates to one or more of the items mentioned before and after "and/or". For instance, a phrase "item 1 and/or item 2" and similar phrases may relate to one or more of item 1 and item 2. The term "comprising" may in an embodiment refer to "consisting of" but may in another embodiment also refer to "containing at least the defined species and optionally one or more other species".

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The devices, apparatus, or systems may herein amongst others be described during operation. As will be clear to the person skilled in the art, the invention is not limited to methods of operation, or devices, apparatus, or systems in operation.

It should be noted that the above-mentioned embodiments illustrate rather than limit the invention, and that those skilled in the art will be able to design many alternative embodiments without departing from the scope of the appended claims.

In the claims, any reference signs placed between parentheses shall not be construed as limiting the claim.

Use of the verb "to comprise" and its conjugations does not exclude the presence of elements or steps other than those stated in a claim. Unless the context clearly requires otherwise, throughout the description and the claims, the words "comprise", "comprising", and the like are to be construed in an inclusive sense as opposed to an exclusive or exhaustive sense; that is to say, in the sense of "including, but not limited to".

The article "a" or "an" preceding an element does not exclude the presence of a plurality of such elements.

The invention may be implemented by means of hardware comprising several distinct elements, and by means of a suitably programmed computer. In a device claim, or an apparatus claim, or a system claim, enumerating several means, several of these means may be embodied by one and the same item of hardware. The mere fact that certain measures are recited in mutually different dependent claims does not indicate that a combination of these measures cannot be used to advantage. In yet a further aspect, the invention (thus) provides a software product, which, when running on a computer is capable of bringing about (one or more embodiments of) the method as described herein.

The invention also provides a control system that may control the device, apparatus, or system, or that may execute the herein described method or process. Yet further, the invention also provides a computer program product, when running on a computer which is functionally coupled to or comprised by the device, apparatus, or system, controls one or more controllable elements of such device, apparatus, or system.

The invention further applies to a device, apparatus, or system comprising one or more of the characterizing features described in the description and/or shown in the attached drawings. The invention further pertains to a method or process comprising one or more of the characterizing features described in the description and/or shown in the attached drawings.

## Claims

1. A light generating system (1000) comprising n1 light generating devices (100) and optics (400), wherein:
- the n1 light generating devices (100) are configured to generate device light (101) having a wavelength selected from the wavelength range of 100 to 380 nm; wherein n1≥1; wherein the n1 light generating devices (100) comprise one or more laser devices;
- the n1 light generating devices (100) and optics (400) are configured to generate k1 beams (105) of device light (101), wherein each beam (105) has a first beam angle (θ1) and perpendicular thereto a second beam angle (θ2), wherein the first beam angle (θ1) and the second beam angle (θ2) are defined by a full width half maximum of the beam (105), wherein one of the first beam angle (θ1) and the second beam angle (θ2) is selected from the range of at least 5° and the other one of the first beam angle (θ1) and the second beam angle (θ2) is selected from the range of at maximum 2°; wherein k1≥1; and
- the light generating system (1000) is configured to generate system light (1001) comprising at least one beam (105) of device light (101);
- wherein the n1 light generating devices (100) and optics (400) are configured to generate a radially configured beam (105) of device light (101).

2. The light generating system (1000) according to claim 1, wherein the n1 light generating devices (100) comprise (i) one or more first light sources (10) configured to generate infrared or visible first light source light (11) and (ii) an upconverter material (210), wherein the one or more first light sources (10) and the upconverter material (210) are configured to generate the device light (101) having a wavelength in the wavelength range of 100 to 380 nm.

3. The light generating system (1000) according to any one of the preceding claims, wherein the n1 light generating devices (100) comprise one or more second light sources (20) configured to generate second light source light (21) having a wavelength in the wavelength range of 100 to 380 nm, wherein the device light (101) comprises the second light source light (21).

4. The light generating system (1000) according to any one of the preceding claims, wherein two or more light generating devices (100) are configured to generate device light (101) having different centroid wavelengths, having a centroid wavelength difference of at least 15 nm.

5. The light generating system (1000) according to any one of the preceding claims, wherein two or more light generating devices (100) are configured to generate device light (101) in different wavelength ranges selected from a UV-A wavelength range, a UV-B wavelength range, a Near UV-C wavelength range, and a Far UV-C wavelength range.

6. The light generating system (1000) according to any one of the preceding claims, wherein k1 ≥2, wherein each k1 beam (105) has an optical axis (Ok), wherein the k1 optical axes (Ok) have third angles (θ3) relative to a virtual plane, wherein the third angles (θ3) are selected from the range of 0-15°.

7. The light generating system (1000) according to any one of the preceding claims, wherein two or more light generating devices (100) are configured such that the k1 beams (105) have parallel first beam angles (θ1); wherein one of the first beam angle (θ1) and the second beam angle (θ2) is selected from the range of at least 10° and the other one of the first beam angle (θ1) and the second beam angle (θ2) is selected from the range of at maximum 1.5°; and wherein k1=n1; wherein the light generating system (1000) comprises a light exit (1005), via which the system light (1001) escapes from the system (1000), where at the light exit (1005) beam widths (W2) of the k1 beams (105) defined by the first beam angles (θ1) are at least 1 cm.

8. The light generating system (1000) according to any one of the preceding claims, wherein k1≥5, wherein the light generating system (1000) is configured to generate an N*M array of the beams (105) of device light (101), wherein N≥2 and wherein M≥2.

9. The light generating system (1000) according to any one of the preceding claims, wherein the optics (400) comprises (i) first optics (410), wherein the first optics (410) comprise lenses configured to collimate the device light (101), and (ii) second optics (420), wherein the second optics (420) comprise one or more of diffractive optics and refractive optics, configured to impose the shape of the beams (105) with a higher aspect ratio than upstream of the second optics (420); and wherein the laser devices comprise laser diodes.

10. The light generating system (1000) according to any one of the preceding claims, wherein the optics (400) comprises a conical specular reflector.

11. The light generating system (1000) according to any one of the preceding claims, further comprising a control system (300), wherein the control system (300) is configured to control one or more of (i) the n1 light generating devices (100), and (ii) optional controllable optics (430), wherein the optional controllable optics (430) are configured to control a propagation direction of at least one of the k1 beams (105).

12. The light generating system according to any one of the preceding claims, further comprising a second light generating device (120), wherein the second light generating device (120) is configured to generate violet second device light (121) having a wavelength selected from the 380-440 nm wavelength range; wherein the second light generating device (120) and the optics (400) are configured to generate a second beam (106) of second device light, wherein the second beam (106) has a first beam angle (θ21) and perpendicular thereto a second beam angle (θ22), wherein the first beam angle (θ21) and the second beam angle (θ22) are defined by a full width half maximum of the second beam (106), wherein one of the first beam angle (θ21) and the second beam angle (θ22) is selected from the range of at least 5° and the other one of the first beam angle (θ21) and the second beam angle (θ22) is selected from the range of at maximum 2°; and wherein the second light generating device (120) comprises a laser diode.

13. The light generating system (1000) according to any one of the preceding claims 4, 5, and 12, wherein the optics (400) comprises one or more of a reflective polarizer and UV dichroic mirror configured to combine beams (105,106) of device light (101,121) having different spectral power distributions.

14. A method for treating a gas or a surface in a space (1300), wherein the space (1300) is external from the light generating system (1000) according to any one of the preceding claims 1-13, the method comprising providing system light (1001) to the gas or the surface with the light generating system (1000).

15. The method according to claim 14, comprising: providing system light (1001) in the space (1300) such that undisturbed beams (105) are only available in a space part h1 meters above a floor in the space (1300), wherein h1 is selected from the range of at least 220 cm; wherein the k1 beams (105) have optical axes (Ok), wherein the optical axes (Ok) have an angle (θ4) with a horizontal selected from the range of larger than 0° and equal to or smaller than 15°, and wherein the optical axes (Ok) are directed away from the floor.

## Patentansprüche

1. Lichterzeugungssystem (1000), umfassend n1 Lichterzeugungsvorrichtungen (100) und Optiken (400), wobei:
- die n1 Lichterzeugungsvorrichtungen (100) konfiguriert sind, um Vorrichtungslicht (101) zu erzeugen, das eine Wellenlänge aufweist, die aus dem Wellenlängenbereich von 100 bis 380 nm ausgewählt ist; wobei n1≥1; wobei die n1 Lichterzeugungsvorrichtungen (100) eine oder mehrere Laservorrichtungen umfassen;
- die n1 Lichterzeugungsvorrichtungen (100) und Optiken (400) konfiguriert sind, um k1 Strahlen (105) von Vorrichtungslicht (101) zu erzeugen, wobei jeder Strahl (105) einen ersten Strahlwinkel (θ1) und senkrecht dazu einen zweiten Strahlwinkel (θ2) aufweist, wobei der erste Strahlwinkel (θ1) und der zweite Strahlwinkel (θ2) durch eine Halbwertsbreite des Strahls (105) definiert sind, wobei einer des ersten Strahlwinkels (θ1) und des zweiten Strahlwinkels (θ2) aus dem Bereich von mindestens 5° ausgewählt ist und der andere des ersten Strahlwinkels (θ1) und des zweiten Strahlwinkels (θ2) aus dem Bereich von höchstens 2° ausgewählt ist; wobei k1≥1; und
- das Lichterzeugungssystem (1000) konfiguriert ist, um Systemlicht (1001) zu erzeugen, umfassend mindestens einen Strahl (105) von Vorrichtungslicht (101);
- wobei die n1 Lichterzeugungsvorrichtungen (100) und Optiken (400) konfiguriert sind, um einen radial konfigurierten Strahl (105) von Vorrichtungslicht (101) zu erzeugen.

2. Lichterzeugungssystem (1000) nach Anspruch 1, wobei die n1 Lichterzeugungsvorrichtungen (100) (i) eine oder mehrere erste Lichtquellen (10), die konfiguriert sind, um infrarotes oder sichtbares erstes Lichtquellenlicht (11) zu erzeugen, und (ii) ein Aufkonvertermaterial (210) umfassen, wobei die eine oder die mehreren ersten Lichtquellen (10) und das Aufkonvertermaterial (210) konfiguriert sind, um das Vorrichtungslicht (101) zu erzeugen, das eine Wellenlänge in dem Wellenlängenbereich von 100 bis 380 nm aufweist.

3. Lichterzeugungssystem (1000) nach einem der vorstehenden Ansprüche, wobei die n1 Lichterzeugungsvorrichtungen (100) eine oder mehrere zweite Lichtquellen (20) umfassen, die konfiguriert sind, um zweites Lichtquellenlicht (21) zu erzeugen, das eine Wellenlänge in dem Wellenlängenbereich von 100 bis 380 nm aufweist, wobei das Vorrichtungslicht (101) das zweite Lichtquellenlicht (21) umfasst.

4. Lichterzeugungssystem (1000) nach einem der vorstehenden Ansprüche, wobei zwei oder mehr Lichterzeugungsvorrichtungen (100) konfiguriert sind, um Vorrichtungslicht (101) zu erzeugen, das unterschiedliche Schwerpunktwellenlängen aufweist, die einen Schwerpunktwellenlängenunterschied von mindestens 15 nm aufweisen.

5. Lichterzeugungssystem (1000) nach einem der vorstehenden Ansprüche, wobei zwei oder mehr Lichterzeugungsvorrichtungen (100) konfiguriert sind, um Vorrichtungslicht (101) in unterschiedlichen Wellenlängenbereichen zu erzeugen, die aus einem UV-A-Wellenlängenbereich, einem UV-B-Wellenlängenbereich, einem Nah-UV-C-Wellenlängenbereich und einem Fern-UV-C-Wellenlängenbereich ausgewählt sind.

6. Lichterzeugungssystem (1000) nach einem der vorstehenden Ansprüche, wobei k1≥2, wobei jeder k1 Strahl (105) eine optische Achse (Ok) aufweist, wobei die k1 optischen Achsen (Ok) dritte Winkel (θ3) relativ zu einer virtuellen Ebene aufweisen, wobei die dritten Winkel (θ3) aus dem Bereich von 0-15° ausgewählt sind.

7. Lichterzeugungssystem (1000) nach einem der vorstehenden Ansprüche, wobei zwei oder mehr Lichterzeugungsvorrichtungen (100) derart konfiguriert sind, dass die k1 Strahlen (105) parallele erste Strahlwinkel (θ1) aufweisen; wobei einer des ersten Strahlwinkels (θ1) und des zweiten Strahlwinkels (θ2) aus dem Bereich von mindestens 10° ausgewählt ist und der andere des ersten Strahlwinkels (θ1) und des zweiten Strahlwinkels (θ2) aus dem Bereich von höchstens 1,5° ausgewählt ist; und wobei k1=n1; wobei das Lichterzeugungssystem (1000) einen Lichtausgang (1005) umfasst, über den das Systemlicht (1001) aus dem System (1000) austritt, wobei an dem Lichtausgang (1005) Strahlbreiten (W2) der k1 Strahlen (105), die durch die ersten Strahlwinkel (θ1) definiert sind, mindestens 1 cm betragen.

8. Lichterzeugungssystem (1000) nach einem der vorstehenden Ansprüche, wobei k1≥5, wobei das Lichterzeugungssystem (1000) konfiguriert ist, um eine N*M-Anordnung der Strahlen (105) von Vorrichtungslicht (101) zu erzeugen, wobei N≥2 und wobei M≥2.

9. Lichterzeugungssystem (1000) nach einem der vorstehenden Ansprüche, wobei die Optiken (400) (i) erste Optiken (410), wobei die ersten Optiken (410) Linsen umfassen, die konfiguriert sind, um das Vorrichtungslicht (101) zu kollimieren, und (ii) zweite Optiken (420) umfassen, wobei die zweiten Optiken (420) eine oder mehrere von diffraktiven Optiken und refraktiven Optiken umfassen, die konfiguriert sind, um die Form der Strahlen (105) mit einem höheren Seitenverhältnis als stromaufwärts der zweiten Optiken (420) anzuordnen; und wobei die Laservorrichtungen Laserdioden umfassen.

10. Lichterzeugungssystem (1000) nach einem der vorstehenden Ansprüche, wobei die Optiken (400) einen konischen Spiegelreflektor umfassen.

11. Lichterzeugungssystem (1000) nach einem der vorstehenden Ansprüche, ferner umfassend ein Steuersystem (300), wobei das Steuersystem (300) konfiguriert ist, um eines oder mehrere von (i) den n1 Lichterzeugungsvorrichtungen (100) und (ii) optionalen steuerbaren Optiken (430) zu steuern, wobei die optionalen steuerbaren Optiken (430) konfiguriert sind, um eine Ausbreitungsrichtung mindestens eines der k1 Strahlen (105) zu steuern.

12. Lichterzeugungssystem nach einem der vorstehenden Ansprüche, ferner umfassend eine zweite Lichterzeugungsvorrichtung (120), wobei die zweite Lichterzeugungsvorrichtung (120) konfiguriert ist, um violettes zweites Vorrichtungslicht (121) zu erzeugen, das eine Wellenlänge aufweist, die aus dem Wellenlängenbereich von 380-440 nm ausgewählt ist; wobei die zweite Lichterzeugungsvorrichtung (120) und die Optiken (400) konfiguriert sind, um einen zweiten Strahl (106) aus zweitem Vorrichtungslicht zu erzeugen, wobei der zweite Strahl (106) einen ersten Strahlwinkel (θ21) und senkrecht dazu einen zweiten Strahlwinkel (θ22) aufweist, wobei der erste Strahlwinkel (θ21) und der zweite Strahlwinkel (θ22) durch eine Halbwertsbreite des zweiten Strahls (106) definiert sind, wobei einer des ersten Strahlwinkels (θ21) und des zweiten Strahlwinkels (θ22) aus dem Bereich von mindestens 5° ausgewählt ist und der andere des ersten Strahlwinkels (θ21) und des zweiten Strahlwinkels (θ22) aus dem Bereich von höchstens 2° ausgewählt ist; und wobei die zweite Lichterzeugungsvorrichtung (120) eine Laserdiode umfasst.

13. Lichterzeugungssystem (1000) nach einem der vorstehenden Ansprüche 4, 5 und 12, wobei die Optiken (400) einen oder mehrere von einem reflektierenden Polarisator und einem UV-dichroitischen Spiegel umfassen, die konfiguriert sind, um Strahlen (105, 106) von Vorrichtungslicht (101, 121), die unterschiedliche spektrale Leistungsverteilungen aufweisen, zu kombinieren.

14. Verfahren zum Behandeln eines Gases oder einer Oberfläche in einem Raum (1300), wobei der Raum (1300) extern zu dem Lichterzeugungssystem (1000) nach einem der vorstehenden Ansprüche 1 bis 13 ist, das Verfahren umfassend das Bereitstellen von Systemlicht (1001) zu dem Gas oder der Oberfläche mit dem Lichterzeugungssystem (1000).

15. Verfahren nach Anspruch 14, umfassend: derartiges Bereitstellen von Systemlicht (1001) in dem Raum (1300), dass ungestörte Strahlen (105) nur in einem Raumteil h1 Meter über einem Boden in dem Raum (1300) verfügbar sind, wobei h1 aus dem Bereich von mindestens 220 cm ausgewählt ist; wobei die k1 Strahlen (105) optische Achsen (Ok) aufweisen, wobei die optischen Achsen (Ok) einen Winkel (θ4) mit einer Horizontalen aufweisen, der aus dem Bereich von größer als 0° und gleich oder kleiner als 15° ausgewählt ist, und wobei die optischen Achsen (Ok) von dem Boden weg gerichtet sind.

## Revendications

1. Système générateur de lumière (1000) comprenant n1 dispositifs générateurs de lumière (100) et des optiques (400), dans lequel :
- les n1 dispositifs générateurs de lumière (100) sont configurés pour générer de la lumière de dispositif (101) ayant une longueur d'onde sélectionnée parmi la plage de longueur d'onde de 100 à 380 nm ; dans lequel n1≥1 ; dans lequel les n1 dispositifs générateurs de lumière (100) comprennent un ou plusieurs dispositifs laser ;
- les n1 dispositifs générateurs de lumière (100) et les optiques (400) sont configurés pour générer k1 faisceaux (105) de lumière de dispositif (101), dans lequel chaque faisceau (105) a un premier angle de faisceau (θ1) et perpendiculaire à celui-ci un deuxième angle de faisceau (θ2), dans lequel le premier angle de faisceau (θ1) et le deuxième angle de faisceau (θ2) sont définis par une largeur totale à mi-hauteur du faisceau (105), dans lequel l'un du premier angle de faisceau (θ1) et du deuxième angle de faisceau (θ2) est sélectionné dans la plage d'au moins 5° et l'autre du premier angle de faisceau (θ1) et du deuxième angle de faisceau (θ2) est sélectionné dans la plage d'au maximum 2° ; dans lequel k1≥1 ; et
- le système générateur de lumière (1000) est configuré pour générer de la lumière de système (1001) comprenant au moins un faisceau (105) de lumière de dispositif (101) ;
- dans lequel les n1 dispositifs générateurs de lumière (100) et les optiques (400) sont configurés pour générer un faisceau (105) configuré radialement de lumière de dispositif (101).

2. Système générateur de lumière (1000) selon la revendication 1, dans lequel les n1 dispositifs générateurs de lumière (100) comprennent (i) une ou plusieurs premières sources de lumière (10) configurées pour générer de la lumière de première source de lumière (11) infrarouge ou visible et (ii) un matériau convertisseur ascendant (210), dans lequel la ou les premières sources de lumière (10) et le matériau convertisseur ascendant (210) sont configurés pour générer la lumière de dispositif (101) ayant une longueur d'onde dans la plage de longueur d'onde de 100 à 380 nm.

3. Système générateur de lumière (1000) selon l'une quelconque des revendications précédentes, dans lequel les n1 dispositifs générateurs de lumière (100) comprennent une ou plusieurs secondes sources de lumière (20) configurées pour générer de la lumière de seconde source de lumière (21) ayant une longueur d'onde dans la plage de longueur d'onde de 100 à 380 nm, dans lequel la lumière de dispositif (101) comprend la lumière de seconde source de lumière (21).

4. Système générateur de lumière (1000) selon l'une quelconque des revendications précédentes, dans lequel deux dispositifs générateurs de lumière (100) ou plus sont configurés pour générer de la lumière de dispositif (101) ayant des longueurs d'onde centroïdes différentes, ayant une différence de longueur d'onde centroïde d'au moins 15 nm.

5. Système générateur de lumière (1000) selon l'une quelconque des revendications précédentes, dans lequel deux dispositifs générateurs de lumière (100) ou plus sont configurés pour générer de la lumière de dispositif (101) dans des plages de longueur d'onde différentes sélectionnées parmi une plage de longueur d'onde UV-A, une plage de longueur d'onde UV-B, une plage de longueur d'onde UV-C proche, et une plage de longueur d'onde UV-C lointain.

6. Système générateur de lumière (1000) selon l'une quelconque des revendications précédentes, dans lequel k1≥2, dans lequel chacun des k1 faisceaux (105) a un axe optique (Ok), dans lequel les k1 axes optiques (Ok) ont des troisièmes angles (θ3) par rapport à un plan virtuel, dans lequel les troisièmes angles (θ3) sont sélectionnés parmi la plage de 0 à 15°.

7. Système générateur de lumière (1000) selon l'une quelconque des revendications précédentes, dans lequel deux dispositifs générateurs de lumière (100) ou plus sont configurés de sorte que les k1 faisceaux (105) ont des premiers angles de faisceau (θ1) parallèles ; dans lequel l'un du premier angle de faisceau (θ1) et du deuxième angle de faisceau (θ2) est sélectionné dans la plage d'au moins 10° et l'autre du premier angle de faisceau (θ1) et du deuxième angle de faisceau (θ2) est sélectionné dans la plage d'au maximum 1,5° ; et dans lequel k1=n1 ; dans lequel le système générateur de lumière (1000) comprend une sortie de lumière (1005), par l'intermédiaire de laquelle la lumière de système (1001) s'échappe du système (1000), où au niveau de la sortie de lumière (1005) des largeurs de faisceau (W2) des k1 faisceaux (105) définis par les premiers angles de faisceau (θ1) sont d'au moins 1 cm.

8. Système générateur de lumière (1000) selon l'une quelconque des revendications précédentes, dans lequel k1≥5, dans lequel le système générateur de lumière (1000) est configuré pour générer une matrice N*M des faisceaux (105) de lumière de dispositif (101), dans lequel N≥2 et dans lequel M≥2.

9. Système générateur de lumière (1000) selon l'une quelconque des revendications précédentes, dans lequel les optiques (400) comprennent (i) des premières optiques (410), dans lequel les premières optiques (410) comprennent des lentilles configurées pour collimater la lumière de dispositif (101), et (ii) des secondes optiques (420), dans lequel les secondes optiques (420) comprennent un ou plusieurs parmi des optiques diffractives et des optiques réfringentes, configurées pour imposer la forme des faisceaux (105) avec un rapport d'aspect plus élevé qu'en amont des secondes optiques (420) ; et dans lequel les dispositifs laser comprennent des diodes laser.

10. Système générateur de lumière (1000) selon l'une quelconque des revendications précédentes, dans lequel les optiques (400) comprennent un réflecteur spéculaire conique.

11. Système générateur de lumière (1000) selon l'une quelconque des revendications précédentes, comprenant en outre un système de commande (300), dans lequel le système de commande (300) est configuré pour commander un ou plusieurs parmi (i) les n1 dispositifs générateurs de lumière (100), et (ii) des optiques commandables facultatives (430), dans lequel les optiques commandables facultatives (430) sont configurées pour commander une direction de propagation d'au moins l'un des k1 faisceaux (105).

12. Système générateur de lumière selon l'une quelconque des revendications précédentes, comprenant en outre un second dispositif générateur de lumière (120), dans lequel le second dispositif générateur de lumière (120) est configuré pour générer de la lumière violette de second dispositif (121) ayant une longueur d'onde sélectionnée parmi la plage de longueur d'onde de 380 à 440 nm ; dans lequel le second dispositif générateur de lumière (120) et les optiques (400) sont configurés pour générer un second faisceau (106) de lumière de second dispositif, dans lequel le second faisceau (106) a un premier angle de faisceau (θ21) et perpendiculaire à celui-ci un deuxième angle de faisceau (θ22), dans lequel le premier angle de faisceau (θ21) et le deuxième angle de faisceau (θ22) sont définis par une largeur totale à mi-hauteur du second faisceau (106), dans lequel l'un du premier angle de faisceau (θ21) et du deuxième angle de faisceau (θ22) est sélectionné dans la plage d'au moins 5° et l'autre du premier angle de faisceau (θ21) et du deuxième angle de faisceau (θ22) est sélectionné dans la plage d'au maximum 2° ; et dans lequel le second dispositif générateur de lumière (120) comprend une diode laser.

13. Système générateur de lumière (1000) selon l'une quelconque des revendications 4, 5 et 12 précédentes, dans lequel les optiques (400) comprennent un ou plusieurs parmi un polariseur réfléchissant et un miroir dichroïque UV configurés pour combiner des faisceaux (105, 106) de lumière de dispositif (101, 121) ayant des distributions de puissance spectrale différentes.

14. Procédé permettant de traiter un gaz ou une surface dans un espace (1300), dans lequel l'espace (1300) est externe par rapport au système générateur de lumière (1000) selon l'une quelconque des revendications 1 à 13 précédentes, le procédé comprenant la fourniture de lumière de système (1001) au gaz ou à la surface avec le système générateur de lumière (1000).

15. Procédé selon la revendication 14, comprenant : la fourniture de lumière de système (1001) dans l'espace (1300) de telle sorte que des faisceaux non perturbés (105) sont uniquement disponibles dans une partie d'espace à h1 mètres au-dessus d'un sol dans l'espace (1300), dans lequel h1 est sélectionné dans la plage d'au moins 220 cm ; dans lequel les k1 faisceaux (105) ont des axes optiques (Ok), dans lequel les axes optiques (Ok) ont un angle (θ4) avec une horizontale sélectionné dans la plage de plus de 0° et égal ou inférieur à 15°, et dans lequel les axes optiques (Ok) sont dirigés à l'écart du sol.
